# EUROPEAN PATENT SPECIFICATION

(11) **EP 0 408 398 B1**
(45) Date of publication and mention of the grant of the patent: **05.04.1995**
(21) Application number: 90307742.8
(22) Date of filing: 16.07.1990
(51) Int. Cl.: C07C 323/25, B01J 2/20

(54) **Granular cysteamine hydrochloride and method for production thereof**
Granuliertes Cysteamin-Hydrochlorid und Verfahren zu dessen Herstellung
Chlorhydrate de cystéamine en grains et procédé pour sa préparation

(30) Priority: 14.07.1989 JP 180181/89; 14.08.1989 JP 207858/89; 15.08.1989 JP 209597/89; 15.08.1989 JP 209601/89; 11.09.1989 JP 232720/89; 12.09.1989 JP 234733/89; 13.09.1989 JP 235564/89; 14.09.1989 JP 236859/89; 18.09.1989 JP 240169/89; 18.09.1989 JP 240170/89
(43) Date of publication of application: 16.01.1991
(73) Proprietor: NIPPON SHOKUBAI CO., LTD., Chuo-ku, Osaka-shi, Osaka-fu 541 (JP)
(72) Inventor: Yuujirou, Gotou, Kawasaki-shi, Kanagawa-ken (JP); Akira, Tamura, Okayama-ken (JP); Hiromi, Yokoyama, Yokohama-shi, Kanagawa-ken (JP)
(74) Representative: Rees, David Christopher

(56) References cited:
- DE-A- 3 025 461
- DE-A- 3 049 196
- US-A- 4 507 500
- PATENT ABSTRACTS OF JAPAN, unexamined applications, C field, vol. 4, no. 173,November 29, 1980 THE PATENT OFFICE JAPANESE GOVERNMENT page 78 C 32
- PATENT ABSTRACTS OF JAPAN, unexamined applications, C field, vol. 6, no. 244,December 2, 1982 THE PATENT OFFICE JAPANESE GOVERNMENT page 105 C 138
- PATENT ABSTRACTS OF JAPAN, unexamined applications, C field, vol. 6, no. 244,December 2, 1982 THE PATENT OFFICE JAPANESE GOVERNMENT page 105 C 138
- PATENT ABSTRACTS OF JAPAN, unexamined applications, C field, vol. 6, no. 174,September 8, 1982 THE PATENT OFFICE JAPANESE GOVERNMENT page 73 C 123
- PATENT ABSTRACTS OF JAPAN, unexamined applications, C field, vol. 6, no. 139,July28, 1982 THE PATENT OFFICE JAPANESE GOVERNMENT page 143 C 116
- PATENT ABSTRACTS OF JAPAN, unexamined applications, C field, vol. 6, no. 127,Junly 13, 1982 THE PATENT OFFICE JAPANESE GOVERNMENT page 88 C 113
- PATENT ABSTRACTS OF JAPAN, unexamined applications, C field, vol. 7, no. 53,March 3, 1983 THE PATENT OFFICE JAPANESE GOVERNMENT page 26 C 154
- PATENT ABSTRACTS OF JAPAN, unexamined applications, C field, vol. 9, no. 188,August 3, 1985 THE PATENT OFFICE JAPANESE GOVERNMENT page 69 C 295

## Description

### BACKGROUND OF THE INVENTION

### Field of the Invention:

This invention relates to granular cysteamine hydrochloride and a method for the production thereof. More particularly, it relates to granules of cysteamine hydrochloride sparingly productive of very minute particles and highly uniform in particle size, excellent in operational efficiency of handling as evinced by unsusceptibility to agglomeration while in storage, and also excellent in solubility in inorganic acids and organic liquids and a method for the production thereof.

### Description of the Prior Art:

Cysteamine hydrochloride is a useful compound as a raw material for medicines, agricultural pesticides, for example.

Heretofore, the cysteamine hydrochloride has been generally produced by any of the following methods.
(1) A method which produces cysteamine hydrochloride by preparing cysteamine from ethylene imine and hydrogen sulfide and causing hydrogen chloride to react with cysteamine.
(2) A method which forms cysteamine hydrochloride by adding hydrochloric acid to 2-dimethyl-thiazolidine [JP-B-50-29,444(1975)].
(3) A method which forms cysteamine hydrochloride from monoethanolamine as a starting raw material [JP-A-57-88,171(1982), JP-A-57-144,252(1982), JP-A-55-17,019(1980), JP-A-57-64,684(1982), JP-A-57-53,458(1982), JP-A-57-67,555(1982), and JP-A-57-64,661(1982)].

US-A-4 507 500 discloses a process for producing 2-mercaptoethylamine hydrohalides of the general formula 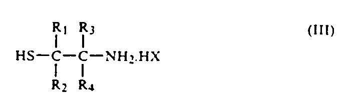
wherein R₁, R₂, R₃ and R₄ are identical or different, and each represents a hydrogen atom, a lower alkyl group, a hydroxy-substituted lower alkyl group or a phenyl group, and X represents a halogen atom,
which comprises reacting a 2-mercaptothiazoline of the general formula 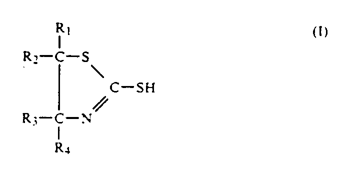
wherein R₁, R₂, R₃ and R₄ are as defined above, with a 2-halogenoethylamine hydrohalide of the general formula 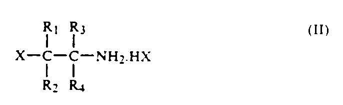
wherein R₁, R₂, R₃, R₄ and X are as defined above, in the presence of water.

Heretofore, the cysteamine hydrochloride has been generally handled in a particulate form. The cysteamine hydrochloride itself is stimulative to the human body. Particularly when this compound is inhaled in the form of fine powder, it stimulates the nasal cavity and the pharynx and incites cough and sneeze. When the powder is left adhering to the skin, it causes inflammation. When the cysteamine hydrochloride in a particulate form containing a fine powder in a large proportion is to be handled, therefore, a serious attention should be paid to protecting the skin against contact with the compound to the fullest possible extent.

Further, the cysteamine hydrochloride in the powdery form has the disadvantage that it agglomerates into lumps during a protracted storage in a container and defies removal from the container and, even when such lumps are managed to be taken out of the container, must be pulverized prior to use. The lumps of cysteamine hydrochloride have another problem of taking up much time in attaining necessary solution.

The cysteamine hydrochloride in the powdery state, deserves well to be called a highly problematic form of product.

The conventional cysteamine hydrochloride which comes in the powdery form has many problems as described above and entails various inconveniences when it is handled in large amounts normal in commerce.

An object of this invention, therefore, is to provide cysteamine hydrochloride which retains the form imparted as a finished product stably for a long time without either appreciably producing a fine powder or undergoing agglomeration while in storage and a method for the production thereof.

These objects are accomplished by granular cysteamine hydrochloride obtained by melting powdery cysteamine hydrochloride and cooling and solidifying the resultant molten cysteamine hydrochloride according to the process disclosed in claim 1 and dependent claims.

The melted granular cysteamine hydrochloride according with the present invention manifests an outstanding effect of avoiding agglomeration while in storage in a container and showing prominent solubility to inorganic acids and organic liquid as compared with the conventional powdery cysteamine hydrochloride. It further has the advantage of not only avoiding the otherwise inevitable possibility of powdery cysteamine hydrochloride stimulating the nasal cavity and the pharynx on inhalation and inducing inflammation of the skin on contact but also preventing the produced granules from substantially absorbing moisture because a polyhydric alcohol-containing solution is used for spray cooling the substrate.

### BRIEF DESCRIPTION OF THE DRAWINGS

Fig. 1 is a schematic cross section illustrating a molding device.
Fig. 2 is a schematic cross section illustrating a granulating device
Fig. 3 is a schematic cross section illustrating a dropping device and
Fig. 4 is a schematic cross section illustrating a dropping device.

### EXPLANATION OF THE PREFERRED EMBODIMENT

The present invention relates to granular cysteamine hydrochloride obtained by melting cysteamine hydrochloride powder and cooling and solidifying the molten cysteamine hydrochloride. It further relates to a method for the production of granular cysteamine hydrochloride, which method comprises melting cysteamine hydrochloride powder and dropping the resultant molten cysteamine hydrochloride onto a corrosionproof substrate retained at a temperature of not higher than 65°C thereby cooling and soldfying the drops of cysteamine hydrochloride.

The granular cysteamine hydrochloride according with the present invention possesses an average particle diameter in the range of 0.1 to 20 mm, preferably 1 to 15 mm.

The cysteamine hydrochloride which can be granulated by the method of this invention assumes a solid state at normal room temperature and possesses a melting point of about 68°C. For the melting of the cysteamine hydrochloride, a temperature required to be higher than the melting point and desired to be in the range of 68° to 150°C, preferably 70° to 100°C is adopted. The heating of the cysteamine hydrochloride is carried out in a corrosionproof container or line provided with heating means such as a jacket, a coil, and/or an electric heater.

Since the cysteamine hydrochloride is highly hygroscopic and very susceptible to oxidation at an elevated temperature, it is desired to be heated in an atmosphere of nitrogen gas. To be solidified, the molten cysteamine hydrochloride is desired to be suddenly cooled by being dropped onto a flat and smooth corrosionproof substrate kept cooled at a temperature falling in the range of 40° to 10°C. Generally, this cooling is effected below the solidifying point for a period not exceeding 30 minutes. The materials which are usable for the corrosionproof substrate include such metals as stainless steel, titanium, and Hastelloy, such resins as Teflon, polypropylene, and polyethylene, and such rubbers as Neoprene and Byton, for example.

For the cooling, a method for cooling the surface of the substrate opposite to the surface exposed to the dropping molten cysteamine hydrochloride specifically by circulation of cooling water may be adopted. The method which comprises melting the cysteamine hydrochloride and cooling and solidifying the molten cysteamine hydrochloride is generally carried out under normal pressure. It may be performed, as occasion demands, under a vacuum or under increased pressure. The granulation of the cysteamine hydrochloride is desired to be attained by cooling and solidifying the molten cysteamine hydrochloride by the use of a plate type dropping granulating device.

The molten cysteamine hydrochloride is supplied to the plate type dropping granulating device. The term "plate type dropping granulating device" as used herein is a general term applied to any of the devices which are adapted to produce granules by dropping a molten substance onto a cooled substrate thereby cooling and solidifying the drops of the molten substance.

In the present invention, the molten cysteamine hydrochloride obtained by heating to a temperature exceeding the melting point is quenched to be solidified and granulated by being dropped onto the corrosionproof substrate of the dropping granulating device kept cooled in the temperature range of 40 to 10°C.

Though the average particle diameter of the granules of cysteamine hydrochloride of this invention is determined by the diameter of the holes for the dropping, the dropping temperature, the temperature of the molten cysteamine hydrochloride, and the speed of movement of the corrosionproof substrate, it is selected in the range of 0.1 to 20 mm, preferably 1 to 15 mm, depending on the handling as during solidification and the solubility of the product during the course of use. The granules have a hemispherical shape or a semiellipsoidal shape.

The belt substrate of the plate type dropping granulating device which is destined to contact the molten cysteamine hydrochloride is flat and smooth and proof against corrosion. The materials which are usable for the belt substrate include such metals as stainless steel, titanium, and Hastelloy, such resins as Teflon, polypropylene, and polyethylene, and such rubbers as Neoprene and Byton, for example.

The substrate of the plate type dropping granulating device is cooled generally by a method which comprises supplying cooling water to the substrate and then cooling the used cooling water with a chiller or a cooling tower before it is put to re-use.

For the purpose of preventing the cysteamine hydrochloride from absorbing moisture, it is desired to have the dropping part and the cooling part of the plate type dropping granulating device or the entire device enveloped with a dehumidified atmosphere of an inert gas such as air or nitrogen.

Though the solidifying time is not specifically defined, it is generally within 30 minutes.

Though the method which comprises melting the cysteamine hydrochloride and cooling and solidifying the molten cysteamine hydrochloride is performed generally under normal pressure, it may be carried out, as occasion demands, under a vacuum or under increased pressure.

The present invention can be applied to cysteamine hydrochloride powder produced by various methods and does not discriminate the cysteamine hydrochloride powder on account of the method employed for its production. The cysteamine hydrochloride powder may be produced, for example, by the following method.

There is a method for producing cysteamine hydrochloride by preparing cysteamine by the reaction of hydrogen sulfide with ethylene imine and then causing cysteamine to react with hydrochloric acid. More specific, hydrogen sulfide is retained in the presence of a solvent under a pressure in the range of 6 to 10 kg/cm²·G, preferably 7 to 10 kg/cm²·G, at a temperature in the range of 0° to 10°C, preferably 0° to 5°C and ethylene imine is continuously added thereto and allowed to react therewith. Then, the resultant reaction mixture is heated at a temperature 60° to 70°C and stripped of hydrogen sulfide. The residue and hydrochloric acid added thereto are cooled to a temperature below 5°C, preferably in the range of 0° to 5°C, to induce precipitation of crystals. The crystals are separated by filtration and dried in an atmosphere of nitrogen gas.

The solvents which are usable in this invention include alcohols such as methanol, ethanol, and apropyl alcohol, ketones such as acetone, and water, for example. Among other solvents mentioned above, methanol or ethanol proves to be particularly desirable.

There is another method for producing cysteamine hydrochloride by causing the sulfuric ester of 2-aminoethanol to react with carbon disulfide in the presence of an alkali hydroxide and then hydrolyzing 2-mercaptothiazoline, the reaction product, with hydrochloric acid. More specifically, in an aqueous solution containing an alkali hydroxide in a concentration in the range of 20 to 40 % by weight, the sulfuric ester of 2-aminoethanol is caused to react with carbon disulfide at a temperature in the range of 20 to 90°C, preferably 30 to 60°C, for a period in the range of 1 to 15 hours, preferably 4 to 6 hours, to form 2-mercaptothiazoline. This 2-mercaptothiazoline is hydrolyzed with hydrochloric acid to give birth to cysteamine hydrochloride.

There is yet another method for producing cysteamine hydrochloride by causing 2-aminoethyl sulfuric ester to react with sodium thiosulfate in the presence of an alkali hydroxide and then hydrolyzing S-(2-aminoethyl)thiosulfate, the reaction product, with hydrochloric acid. To be more specific, the 2-aminoethanol sulfuric ester is caused to react with sodium thiosulfate in an aqueous solution containing an alkali hydroxide in a concentration in the range of 20 to 45 % by weight at a temperature in the range of at least 20°C, preferably at least 50°C, for a period in the range of 1 to 50 hours, preferably 2 to 40 hours so form S-(2-aminoethyl)thiosulfate. This S-(2-aminoethyl)thiosulfate is hydrolyzed with hydrochloric acid to give birth to the cysteamine hydrochloride.

There is still another method for producing cysteamine hydrochloride by causing 2-chloroethylamine hydrochloride to react with 2-mercaptothiazolidine in the presence of a solvent and then hydrolyzing 2-(2′-aminoethylthio)thiazolidine hydrochloride, the reaction product, with hydrochloric acid. To be more specific, 2-cloroethylamine hydrochloride is dissolved in a concentration in the range of 30 to 70 % by weight in a solvent. The resultant solution is caused to react with 2-mercaptothiazolidine at a temperature in the range of at least 20°C, preferably at least 50°C, for a period in the range of 1 to 50 hours, preferably 2 to 40 hours, to form 2-(2′-aminoethylthio)thiazolidine hydrochloride. Then, this reaction product is hydrolyzed with hydrochloric acid. The solvents which are usable in this reaction include water, methanol, ethanol, isopropanol, ect., for example.

There is further a method for producing cysteamine hydrochloride by causing a 2-dialkylthiazoline to react with water and a halogenated hydroacid in the presence of a solvent. To be more specific, the 2-dialkylthiazolidine is hydrolyzed with the halogenated hydroacid in the presence of a solvent, with the pH value adjusted in the range of 1.5 to 5.0, preferably 2 to 4, and the temperature elevated to a level in the range of 40° to 180°C, preferably 100 to 150°C. Then, the hydrolyzate is deprived of the solvent under a vacuum.

The solvents which are usable in this invention include such polar solvents as water and alcohols, for example. Among other solvents mentioned above, water proves to be particularly desirable. The 2-dialkylthiazolidines which are usable in this invention include 2-dimethylthiazolidine and 2-dimethylethyl thiazolidine, for example. Particularly, 2-dimethyl thiazolidine proves to be desirable. The halogenated hydroacids which are usable in the present invention include hydrochloric acid and hydrobromic acid, for example. Particularly, hydrochloric acid proves to be desirable. The cysteamine hydrochlorides which are obtainable in the present invention include aminoethane thiol salt and β-aminoethylmercaptan salt, for example. Particularly, cysteamine hydrochloride (aminoethane thiol hydrochloride) is obtained desirably.

This invention relates to a method for the production of granular cysteamine, which comprises granulating cysteamine hydrochloride by melting the hydrochloride and cooling and solidifying the molten hydrochloride by the use of a plate type dropping granulating device while having the cooling substrate of the plate type dropping granulating device kept cooled by spraying a polyhydric alcohol-containing solution on the lower surface of the substrate.

Now, the accompanying drawings will be disclosed.

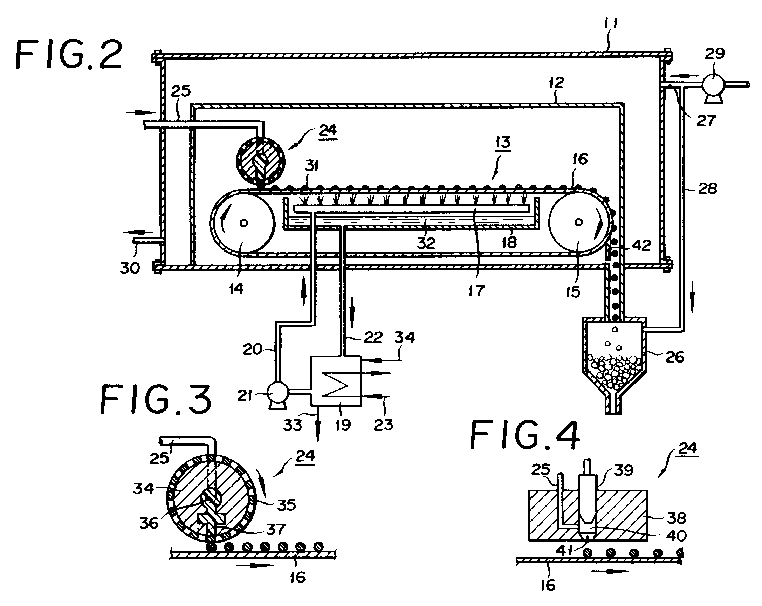

Fig. 2 is a schematic cross section illustrating an apparatus for practising the method of the present invention. Inside a granular granulating chamber 12 installed in a building 11, a conveying device 13 such as, for example, a belt conveyor comprising rolls 14, 15 and an endless belt 16. Below the endless belt 16 is disposed a coolant spraying device 17. Below this spraying device 17 is disposed a coolant recovering device 18. To this spraying device 17, a conduit 20 extended from a cooling device 19 is connected through the medium of a pump 21. To the recovering device 18, a conduit 22 extended from a cooling device 19 is connected. To the cooling device 19, a coolant such as brine is supplied via a conduit 23 to keep the cooling device 19 at a prescribed temperature.

A dropping device 24 for molten cysteamine hydrochloride is disposed above the conveying device 13 and in the nearmost part relative to the direction of motion of the conveyor. To this dropping device 24 is connected a conduit 25 extended from a melting device (not shown) for cysteamine hydrochloride.

A hopper 26 is connected to the lower part of the granulating chamber 12 in the downstream end part relative to the direction of motion of the conveyor. Conduits 27, 28 connected to a dehumidifying device (not shown) are extended through a pump 29 and connected to the interior of the building 11 and the hopper 26. To the opposite side of the building 11, an exhaust gas conduit 30 is connected.

Now, the method for granulating cysteamine hydrochloride by the use of the apparatus constructed as described above will be explained below. First, the cysteamine hydrochloride melted by the melting device (not shown) is supplied to the dropping device 24 through a conduit 25 which is kept warm as occasion demands and caused to fall in drops 31 therefrom onto the belt conveyor 16. The drops 31 deposited on the belt conveyor 16 in motion are cooled and solidified into granules as the coolant spraying device 17 sprays a polyhydric alcohol-containing liquid on the lower surface of the belt conveyor 16. The sprayed polyhydric alcohol-containing liquid, on fulfiling the role of cooling the belt conveyor 16, is recovered by the recovering device 18. Though this recovering device 18 may assume a varying form, it is generally used in the form of a tray. A recovered polyhydric alcohol-containing liquid 32 is forwarded through the conduit 22 to the cooling device 19, cooled therein to a prescribed temperature, and forwarded through the conduit 20 to the coolant spraying device 17 by means of the pump 21.

The granules formed as described above are scraped by a scraper 42 and discharged via the hopper 26. Dry air, when necessary, is introduced through the conduits 27, 28 into the building 11 and the hopper 26 and discharged through the exhaust gas conduit 30, so as to keep the interior of the building 11 and the interior of the hopper 26 in a dry state.

The polyhydric alcohol-containing liquids which are usable in the present invention include ethylene glycol, diethylene glycol, triethylene glycol, tetraethylene glycol, propylene glycol, dipropylene glycol, tripropylene glycol, glycerin, butane diol, neopentyl glycol, and pentaerythritol, for example, which may be used singly, as suitably combined, or as dissolved in water. Among other polyhydric alcohol-containing liquids mentioned above, ethylene glycol, diethylene glycol, and propylene glycol prove to be preferable. Ethylene glycol proves to be most desirable.

The polyhydric alcohol-containing liquid which is supplied to the coolant spraying device absorbs the moisture from within the gas filling the granulating device and consequently serves the purpose of keeping the interior of the granulating device in a dry state. When the polyhydric alcohol-containing liquid is diluted with the absorbed moisture to below a prescribed concentration, is discharged through an outlet 33. A new supply of the polyhydric alcohol-containing liquid is introduced through an inlet 34 by way of replenishment.

The dropping device 24 may be in a varying form. For example, as illustrated in Fig. 3, it comprises a stationary inner tube 34, a porous outer tube 35 disposed coaxially with and rotatably around the inner tube 34, a liquid reservoir 36 excavated near the axis of the inner tube 34 and adapted to receive molten cysteamine hydrochloride supplied through the conduit 25, and a slit 37 formed in the lower part of the inner tube 34 and adapted to drop this molten cysteamine hydrochloride onto the conveyor belt 16. In this case, the molten cysteamine hydrochloride is allowed to fall in drops when the holes in the outer tube 25 coincide with the slit and prevented from falling when the closed wall part of the outer tube 25 is passing the slit.

It is otherwise permissible, as illustrated in Fig. 4, to form a hollow part having a piston 39 inserted through a dropping member proper 38 and allow the conduit 25 to communicate with the hollow part, so that the molten cysteamine hydrochloride will be allowed to fall through lower holes 41 in drops onto the belt conveyor 16 in consequence of the reciprocation of the piston 39.

In this case, the endless belt 16 is made of a metal such as stainless steel, aluminum, or copper and is desired to have the surface thereof coated with fluorine resin. The fluorine resin coating has a thickness generally in the range of 0.001 to 1 mm, preferably 0.001 to 0.5 mm. If this thickness is less than 0.001 mm, the coating is deficient in durability. Conversely, if this thickness exceeds 1 mm, the coating is deficient in thermoconductivity and expensive. The coating may be carried out, for example, by a normal temperature dry spray method and a fluorine resin sticking method. The fluorine resins which are usable for the coating include polytetrafluoroethylene, tetrafluoroethylene-hexafluoropropylene copolymer, polyvinylidene fluoride, polyvinyl fluoride, and tetrafluoroethylene-perfluorovinyl ether copolymer, for example. Among the fluorine resins mentioned above, polytetrafluoroethylene proves to be particularly desirable.

The coating of the fluorine resin mentioned above can be easily formed by impregnating cloth of glass fibers with the fluorine resin, attaching the impregnated glass-fiber cloth fast to an adhesive sheet, and joining the resultant composite to the endless belt of metal through the medium of the adhesive sheet.

Now, the present invention will be described more specifically below with reference to working examples and controls.

### [Method of testing solubility]

In four widemouthed sample vials of glass having an inner volume of 200 cc, 30 g each of a given granular cysteamine hydrochloride was placed and, with the entrapped air displaced with nitrogen, was left standing in a hermetically sealed condition at 20°C for 0 day, 7 days, 14 days, and 30 days before the vials were opened.
(1) In a beaker having an inner volume of 100 ml and held under an atmosphere of nitrogen gas, 40 ml of an aqueous 35 wt% hydrochloric acid solution and 10 g of the granular cysteamine hydrochloride added thereto were mixed with a stirrer at a rotational rate of 200 rpm at 25°C to determine the time required for the granules to dissolve in the aqeous solution.
(2) In a beaker having an inner volume of 100 ml and held under an atmosphere of nitrogen gas, 25 ml of ethanol and 8 g of the granular cysteamine hydrochloride added thereto were mixed with a stirrer at a rotational rate of 200 rpm at 25°C to determine the time required for the granules to dissolve in the ethanol.

### [Method for testing agglomeration]

In four widemouthed sample vials of glass having an inner volume of 200 cc, 30 g each of a given granular cysteamine hydrochloride was placed and, with the entrapped air displaced with nitrogen, was left standing in a hermetically sealed condition at 20°C for 0 day, 7 days, 14 days, and 30 days. Thereafter, the vials were turned upside down and their contents were visually examined as to the condition of motion of particles therein.

### Example 1

In an autoclave having an inner volume of 1 m³, 200 liters of methanol was placed and the air entrapped therein was displaced with nitrogen gas. When 136 g (4 kilomols) of hydrogen sulfide was introduced into the system while in a stirred state with the temperature thereof adjusted in the range of 0° to 5°C, the pressure of the system reached 8.6 kg/cm². With the temperature of the system maintained in the range of 0° to 5°C, a solution of 86 kg (2 kilomols) of ethylene imine in 100 liters of methanol was continuously added for reaction to the system while in a stirred state over a period of 2 hours. After completion of the reaction, the pressure of the system was 3.6 kg/cm² G. The resultant reaction mixture was heated as sealed under an atmosphere of nitrogen gas until a small amount of the methanol was distilled out, to effect expulsion of hydrogen sulfide. It was further cooled to 5°C to induce precipitation of crystals. The crystals were separated by filtration and then dried in an atmosphere of nitrogen gas, to afford white crystalline cysteamine.

The cysteamine thus obtained was transferred into a reaction kettle lined with glass, dissolved in 600 liters of isopropyl alcohol, and then blown with 73 kg of hydrogen chloride gas, to give rise to a cysteamine hydrochloride solution. This solution was cooled to 5°C to induce precipitation of crystals. The crystals were separated by filtration and dried in an atmosphere of nitrogen gas, to obtain white crystalline cysteamine hydrochloride.

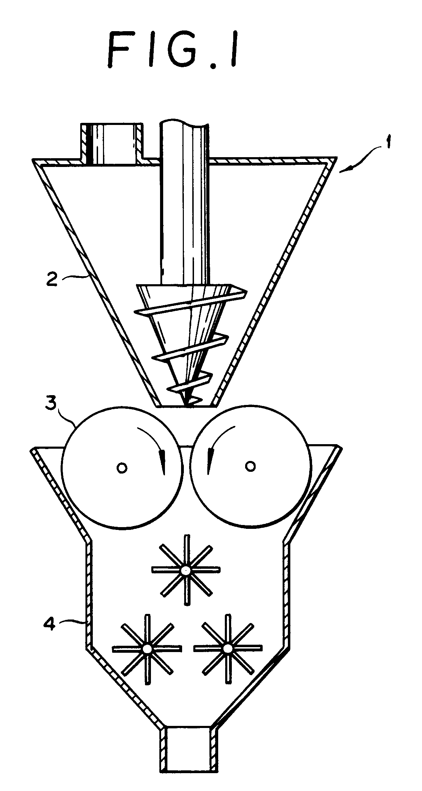

The white crystalline cysteamine hydrochloride powder thus obtained was placed in the hopper 2 of the roll type compressing device (roller compacter) 1 constructed as illustrated in Fig. 1 and compressed with rollers under a roll pressure of 100 atmospheres to produce a sheet. Then, the sheet was treated with the coarse pulverizing and diameter-adjusting device 4. The molded product of cysteamine hydrochloride consequently obtained had particle diameters in the range of 0.5 to 5 mm.

The molded cysteamine hydrochloride was left standing at 30°C for a varying length of time, i.e. 0 to 30 days, and then tested for solubility in an aqueous 35 wt% hydrochloric acid solution and ethanol and for susceptibility to all agglomeration.

The results of the test for solubility are shown in Table 1 and those of the test for agglomeration in Table 2.

**Table 1**

| Number of days of standing | Time for solution (min) in aqueous 35 wt% HCl soln. | Time for solution (min) in ethanol |
|---|---|---|
| 0 | 2.2 | 28 |
| 7 | 2.1 | 28 |
| 14 | 2.1 | 28 |
| 30 | 2.2 | 28 |

**Table 2**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Control 1

In vials having the entrapped air diplaced with nitrogen gas, samples of the same white crystalline cysteamine hydrochloride powder as obtained in Example 1 were left standing at 30°C for varying lengths of time, i.e. 0 to 30 days. After the standing, the samples in their undissolved form were tested for solubility in an aqueous 35 wt% hydrochloric acid solution and ethanol and for susceptibility to agglomeration.

The results of the test for solubility are shown in Table 3 and those of the test for agglomeration in Table 4.

**Table 3**

| Number of days of standing | Time for solution (min) in aqueous 35 wt% HCl soln. | Time for solution (min) in ethanol |
|---|---|---|
| 0 | 2 | 25 |
| 7 | 23 | 68 |
| 14 | 28 | 75 |
| 30 | 30 | 82 |

**Table 4**

| Number of days of standing | Condition of motion |
|---|---|
| 0 | Absence of cohesion between particles |
| 7 | Agglomerated |
| 14 | Agglomerated |
| 30 | Agglomerated |

### Example 2

The same white crystalline cysteamine hydrochloride powder as obtained in Example 1 was dissolved by heating to 80°C, aspirated in a Pasteur Pipet 2 mm in mouth diameter, and dropped onto a plate of polytetrafluoroethylene (marketed under trademark designation of "Teflon") having the opposite side kept at 30°C by cooling with water. The white solid heaps of cysteamine hydrochloride deposited consequently on the Teflon plate were hemispheres 3 to 5 mm in diameter and 2 to 3 mm in height.

The granular cysteamine hydrochloride thus obtained was left standing at 30°C for varying lengths of time, i.e. 0 to 30 days, and then tested for solubility in an aqueous 35 wt% hydrochloric acid solution and ethanol and for susceptibility to agglomeration.

The results of the test for solubility are shown in Table 5 and those of the test for agglomeration in Table 6.

**Table 5**

| Number of days of standing | Time for solution (min) in aqueous 35 wt% HCl soln. | Time for solution (min) in ethanol |
|---|---|---|
| 0 | 2.5 | 32 |
| 7 | 2.4 | 32 |
| 14 | 2.5 | 32 |
| 30 | 2.5 | 32 |

**Table 6**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Example 3

White solid cysteamine hydrochloride hemispheres 10 to 15 mm in diameter and 5 to 6 mm in height obtained as deposited on a polytetrafluoroethylene by following the procedure of Example 2 were tested for solubility in an aqueous 35 wt% hydrochloric acid solution and for susceptibility to agglomeration in the same manner as in Example 1.

The results of the test for solubility are shown in Table 7 and those of the test for agglomeration in Table 8.

**Table 7**

| Number of days of standing | Time for solution (min) in aqueous 35 wt% HCl soln. |
|---|---|
| 0 | 5.4 |
| 7 | 5.5 |
| 14 | 5.4 |
| 30 | 5.4 |

**Table 8**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Example 4

In an autoclave having an inner volume of 1 m², 200 liters of methanol was placed and the air entrapped therein was displaced with nitrogen gas. When 136 kg (4 kilomols) of hydrogen sulfide was introduced into this system while in a stirred state, with the temperature of the system adjusted in the range of 0° to 5°C, the pressure of the system reached 8.6 kg/cm2 G. With the temperature of this system kept in the range of 0° to 5°C, a solution of 86 kg (2 kilomols) of ethylene imine in 100 liters of methanol was continuously added for reaction to the system while in a stirred state over a period of 2 hours. After completion of the reaction, the pressure of the system was 3.6 kg/cm² G.

The resultant reaction mixture was heated as sealed in an atmosphere of nitrogen gas until a small amount of the methanol was distilled out, to effect expulsion of the hydrogen sulfide. The reaction mixture was further cooled to 5°C to induce precipitation of crystals. The crystals were separated by filtration and then dried in an atmosphere of nitrogen gas, to afford white crystalline cysteamine.

The cysteamine thus obtained was transferred into a reaction vessel lined with glass, dissolved in 600 liters of isopropyl alcohol, and then blown with 73 kg of hydrogen chloride gas, to give rise to cysteamine hydrochloride solution. This solution was cooled to 5°C to induce precipitation of crystals. The crystals were separated by filtration and then dried in an atmosphere of nitrogen gas, to produce white crystalline cysteamine hydrochloride.

The white crystalline cysteamine hydrochloride powder thus obtained was melted by heating to about 72°C, supplied to a plate type dropping granulating device, and dropped onto a sheet of polytetrafluoroethylene applied fast to a flat smooth platelike belt of steel having the opposite side thereof cooled with water to 30°C. The white solid heaps of cysteamine hydrochloride consequently deposited on the sheet were hemispheres 3 to 5 mm in diameter and 1 to 3 mm in height.

The granules of cysteamine hydrochloride thus obtained were left standing at 30°C for varying lengths of time, i.e. 0 to 30 days, and then tested for solubility in an aqueous 35 wt% hydrochloric acid solution and ethanol and for susceptibility to agglomeration.

The results of the test for solubility are shown in Table 9 and those of the test for agglomeration in Table 10.

**Table 9**

| Number of days of standing | Time for solution (min) in aqueous 35 wt% HCl soln. | Time for solution (min) in ethanol |
|---|---|---|
| 0 | 1.9 | 25 |
| 7 | 1.8 | 25 |
| 14 | 1.9 | 25 |
| 30 | 1.9 | 25 |

**Table 10**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Example 5

White solid cysteamine hydrochloride hemispheres 10 to 15 mm in diameter and 5 to 6 mm in height obtained by following the procedure of Example 4 were tested for solubility in an aqueous 35 wt% hydrochloric acid solution and for susceptibility to agglomeration.

The results of the test for solubility are shown in Table 11 and those of the test for agglomeration in Table 12.

**Table 11**

| Number of days of standing | Time for solution (min) in aqueous 35 wt% HCl soln. |
|---|---|
| 0 | 4.2 |
| 7 | 4.2 |
| 14 | 4.2 |
| 30 | 4.2 |

**Table 12**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Example 6

The same cysteamine as obtained in Example 1 was transferred into a separable flask of glass, dissolved in 600 ml of isopropyl alcohol, and blown with 73 g of hydrogen chloride gas, to obtain cysteamine hydrochloride solution. This solution was cooled to 5°C to induce precipitation of crystals. The crystals were separated by filtration and dried in an atmosphere of nitrogen and heated to 80°C to be thoroughly dissolved. The solution was aspired with a Pasteur pipet 2 mm in mouth diameter and dropped onto a plate of tetrafluoroethylene having the opposite side thereof cooled with water to 30°C. The white solid cysteamine hydrochloride heaps deposited on the plate were hemispheres 3 to 5 mm in diameter and 2 to 3 mm in height.

The cysteamine hydrochloride hemispheres thus obtained were left standing at 30°C for varying lengths of time, i.e. 0 to 30 days, and then tested for solubility in an aqueous 35 wt% hydrochloric acid solution and ethanol and for susceptibility to agglomeration.

The results of the test for solubility are shown in Table 13 and those of the test for agglomeration in Table 14.

**Table 13**

| Number of days of standing | Time for solution (min) in aqueous 35 wt% HCl soln. | Time for solution (min) in ethanol |
|---|---|---|
| 0 | 2.6 | 32 |
| 7 | 2.4 | 33 |
| 14 | 2.6 | 34 |
| 30 | 2.5 | 33 |

**Table 14**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Example 7

White solid cysteamine hydrochloride hemispheres 10 to 15 mm in diameter and 5 to 6 mm in height obtained by following the procedure of Example 6 were tested for solubility in an aqueous 35 wt% hydrochloric acid solution and for susceptibility to agglomeration in the same manner as in Example 1.

The results of the test for solubility are shown in Table 15 and those of the test for agglomeration in Table 16.

**Table 15**

| Number of days of standing | Time for solution (min) in aqueous 35 wt% HCl soln. |
|---|---|
| 0 | 5.4 |
| 7 | 5.6 |
| 14 | 5.4 |
| 30 | 5.5 |

**Table 16**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Example 8

In a reaction vessel provided with a stirrer, a temperature-regulating device, a refluxing device, and a dropping funnel, 0.1 mol of sulfuric ester of 2-aminoethanol was dissolved at room temperature in an aqueous 20 wt% sodium hydroxide solution containing 0.2 mol of sodium hydroxide. The solution and 0.2 mol of carbon disulfide added thereto were thoroughly stirred, gradually heated to the neighborhood of the boiling point of carbon disulfide, and left reacting at that temperature for 2 hours. Then, the resultant reaction mixture and an ageous 20 wt% sodium hydroxide solution added thereto in an amount of 0.2 mol as sodium hydroxide were left reacting further for 3 hours. The reaction mixture, during the secondary reaction, allowed occurrence of relux because of a slight residue of carbon disulfide. The residue was completely consumed, however, in the latter half phase of the reaction. Finally, the temperature was raised to 60°C to terminate the reaction. Then, the reaction mixture was hot filtered at a temperature above 40°C. The crude crystals consequently obtained was thoroughly washed with cold water, to obtain white 2-mercaptothiazoline crystals. The purity of the product was 99.4% and the yield of the product based on the supplied 2-aminoethanol sulfiric ester was 93.3%.

In a glass autoclave having an inner volume of 1 liter, 119.3 g (1.0 mol) of 2-mercaptothiazoline and 650 cc (4.23 mols) of 21 wet 21 wt% hydrochloric acid were refluxed at 102°C under a pressure of 2.5 kg/cm2 G for 45 hours. After the reaction was stopped, the reaction mixture was gradually reverted to normal pressure and treated with a rotary evaporator for 1 hour and heated under a vacuum at 80oC for 1 hour to effect thorough expulsion of hydrogen chloride and water and concentration to dryness.

Subsequently, the recrystallized concentrate and 60 cc of methanol (purity 99.9%) added thereto as a solvent were heated and stirred for thorough solution. The resultant solution was amply stirred and cooled to 5°C induce precipitation of crystals. The crystals were separated by suction filtration. The produced crystals of cysteamine hydrochloride were dried under a vacuum at 40°C for 2 hours and then thoroughly dissolved by heating to a temperature above 80°C. The solution was aspired with a Pasteur pipet 2 mm in mouth diameter and dropped onto a tetrafluoroethylene plate having the opposite side thereof cooled with water to 30°C. The white solid cysteamine hydrochloride heaps consequently deposited on the plate were hemispheres 3 to 5 mm in diameter and 2 to 3 mm in height.

The hemispheres of cysteamine hydrochloride thus obtained were left standing at 30°C for varying lengths of time, i.e. 0 to 30 days, and then tested for solubility in an aqueous 35 wt% hydrochloric acid solution and ethanol and for susceptibility to agglomeration.

The results of the test for solubility are shown in Table 17 and those of the test for agglomeration in Table 18.

**Table 17**

| Number of days of standing | Time for solution (min) in aqueous 35 wt% HCl soln. | Time for solution (min) in ethanol |
|---|---|---|
| 0 | 2.3 | 31 |
| 7 | 2.3 | 31 |
| 14 | 2.4 | 30 |
| 30 | 2.3 | 32 |

**Table 18**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Control 2

In vials having the trapped air therein displaced with nitrogen, samples of the same white crystalline cysteamine hydrochloride powder as obtained in Example 8 were left standing at 30°C for varying lengths of time, i.e. 0 to 30 days, and were tested in their undissolved state to solubility in an aqueous 35 wt% hydrochloric acid solution and ethanol and for susceptibility to agglomeration.

The results of the test for solubility are shown in Table 19 and those of the test for agglomeration in Table 20.

**Table 19**

| Number of days of standing | Time for solution (min) in aqueous 35 wt% HCl soln. | Time for solution (min) in ethanol |
|---|---|---|
| 0 | 3 | 27 |
| 7 | 25 | 70 |
| 14 | 30 | 75 |
| 30 | 33 | 85 |

**Table 20**

| Number of days of standing | Condition of motion |
|---|---|
| 0 | Absence of cohesion between particles |
| 7 | Agglomerated |
| 14 | Agglomerated |
| 30 | Agglomerated |

### Example 9

In a four-neck flask having an inner volume of 100 ml and provided with a stirrer, a thermometer, a reflux condenser, and a nitrogen gas inlet, 14.1 g (0.1 mol) of 2-aminoethyl sulfuric ester, 16.3 g (0.1 mol) of sodium thiosulfate, and 30 g of water were placed and the resultant reaction solution was adjusted to a pH of about 9.0 by addition of sodium hydroxide. The reaction solution was heated and stirred at a refluxing temperature for 20 hours as gently swept with nitrogen gas. After completion of the reaction, the reaction solution assumed a pH of about 6.5. This reaction solution was filtered to remove the greater part of sodium sulfate. The filtrate was concentrated under a vacuum to dryness. On analysis with an IR and a H-NMR, the product was identified to be practically pure (100%) S-(2-aminoethyl )thiosulfate.

In a four-neck flask provided with a stirrer, a thermometer, a reflux condenser, and a dropping funnel, 50 g of 20 wt% sulfuric acid was placed. In the dropping funnel, 78.5 g of an aqueous 40 wt% S-(2-aminoethyl)thiosulfate solution was placed. The flask was heated in an oil bath to keep the inner temperature thereof in the range of 100° to 105°C and the aqueous thiosulfate solution was added dropwise to the sulfuric acid from the dropping funnel over a period of about 2 hours. The resultant reaction solution was cooled to room temperature, diluted with water, and treated with an anion-exchange resin, Amberlite IRA 400. Then, hydrochloric acid was added to the reaction solution until the pH value reached 4. The resultant solution was concentrated to dryness under a vacuum and subsequently dried under a vacuum, to obtain 25.3 g of powder. When this powder was assayed by iodometry for determination of the SH group, it was found to be cysteamine hydrochloride having a purity of 96.3% (yield 94.2). When this powder was assayed by the thin-layer chromatography, the presence of a very feeble spot of bis(2-aminoethyl)disulfide was recognized. This cysteamine hydrochloride powder was recrystallized from isopropanol, dried under a vacuum, and then dissolved thoroughly by heating to a temperature above 80°C. The resultant solution was aspired with a Pasteur pipet 2 mm in mouth diameter and dropped onto a tetrafluoroethylene plate having the opposite side thereof cooled with water to 30°C. The white solid cysteamine hydrochloride heaps consequently deposited on the plate were hemispheres 3 to 5 mm in diameter and 2 to 3 mm in height.

Samples of the granules of the cysteamine hydrochloride thus obtained were left standing at 30°C for a varying lengths of time, i.e. 0 to 30 days, and then tested for solubility in an aqueous 35 wt% hydrochloric acid solution and ethanol and for susceptibility to agglomeration.

The results of the test for solubility are shown in Table 21 and those of the test for agglomeration in Table 22.

**Table 21**

| Number of days of standing | Time for solution (min) in aqueous 35 wt% HCl soln. | Time for solution (min) in ethanol |
|---|---|---|
| 0 | 2.8 | 34 |
| 7 | 2.8 | 34 |
| 14 | 2.7 | 34 |
| 30 | 2.7 | 34 |

**Table 22**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Control 3

In vials having the entrapped air displaced with nitrogen gas, samples of the same white crystalline cysteamine hydrochloride powder as obtained in Example 9 were left standing at 30°C for varying lengths of time, i.e. 0 to 30 days, and then tested for solubility in an aqueous 35 wt% hydrochloric acid solution and ethanol and for susceptibility to agglomeration.

The results of the test for solubility are shown in Table 23 and those of the test for agglomeration in Table 24.

**Table 23**

| Number of days of standing | Time for solution (min) in aqueous 35 wt% HCl soln. | Time for solution (min) in ethanol |
|---|---|---|
| 0 | 3 | 24 |
| 7 | 26 | 66 |
| 14 | 28 | 75 |
| 30 | 35 | 81 |

**Table 24**

| Number of days of standing | Condition of motion |
|---|---|
| 0 | Absence of cohesion between particles |
| 7 | Agglomerated |
| 14 | Agglomerated |
| 30 | Agglomerated |

### Example 10

In 100 ml of benzene, 0.1 mol of 2-chloroethylamine hydrochloride and 0.1 mol of 2-mercaptothiazoline were dispersed, gradually heated to 80°C, and stirred for reaction at this temperature for 30 minutes. After completion of the reaction, the reaction mixture was filtered to obtain white 2-(2-aminoethylthio)-thiazoline dihydrochloride powder in a yield of 99.7%. In 100 ml of 36 wt% hydrochloric acid, 23.5 g (0.1 mol) of 2-(2-aminoethylthio)-thiazoline dihydrochloride was dissolved. The resultant solution was refluxed for 30 hours, to obtain cysteamine hydrochloride. Then, the cysteamine hydrochloride was concentrated to dryness under a vacuum and then thoroughly dissolved by heating to a temperature above 80°C. The solution was aspired with a Pasteur pipet 2 mm in mouth diameter and dropped onto a tetrafluoroethylene plate having the opposite side thereof cooled with water to 30°C. The white solid cysteamine hydrochloride heaps consequently deposited on the plate were hemispheres 3 to 5 mm in diameter and 2 to 3 mm in height.

The cysteamine hydrochloride hemispheres thus obtained were left standing at 30°C for a varying lengths of time, i.e. 0 to 30 days, and then tested for solubility in an aqueous 35 wt% hydrochloric acid solution and ethanol and for susceptibility to agglomeration.

The results of the test for solubility are shown in Table 25 and those of the test for agglomeration in Table 26.

**Table 25**

| Number of days of standing | Time for solution (min) in aqueous 35 wt% HCl soln. | Time for solution (min) in ethanol |
|---|---|---|
| 0 | 2.7 | 34 |
| 7 | 2.5 | 32 |
| 14 | 2.6 | 33 |
| 30 | 2.5 | 33 |

**Table 26**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Control 4

In vials having the entrapped air therein displaced with nitrogen gas, samples of the same white crystalline cysteamine hydrochloride powder as obtained in Example 10 were left standing at 30°C for varying lengths of time, i.e. 0 to 30 days, and then, in their undissolved state, tested for solubility in an aqueous 35 wt% hydrochloric acid solution and ethanol and for susceptibility to agglomeration.

The results of the test for solubility are shown in Table 27 and those of the test for agglomeration in Table 28.

**Table 27**

| Number of days of standing | Time for solution (min) in aqueous 35 wt% HCl soln. | Time for solution (min) in ethanol |
|---|---|---|
| 0 | 2 | 25 |
| 7 | 24 | 70 |
| 14 | 25 | 70 |
| 30 | 30 | 83 |

**Table 28**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Example 11

In a four-neck flask having an inner volume of 5 liters, 420 g of water was placed and the air entrapped in the flask was displaced with nitrogen gas. Then, the water and 1130 g of 2-dimethylthiazoline added thereto were stirred and heated at a temperature in the range of 40° to 60°C and, in the meantime, adjusted to a pH value of 3.5 by dropwise addition thereto of 35 wt% hydrochloric acid over a period of about 4 hours. Then, the resultant reaction solution was heated under normal pressure to 120°C, then vacuumized to effect removal of acetone and water, reverted to normal pressure with nitrogen gas, and cooled to 80°C.

The molten cysteamine hydrochloride consequently obtained was aspired with a Pasteur pipet 2 mm in mouth diameter and dropped onto a tetrafluoroethylene plate having the opposite side thereof cooled with water to 30°C. The white solid cysteamine hydrochloride heaps consequently deposited on the plate were hemispheres 3 to 5 mm in diameter and 1 to 3 mm in height.

Samples of the cysteamine hydrochloride hemispheres obtained as described above were left standing at 30°C for varying lengths of time, i.e. 0 to 30 days, and tested for solubility in an aqueous 35 wt% hydrochloric acid solution and ethanol and for susceptibility to agglomeration.

The results of the test for solubility are shown in Table 29 and those of the test for agglomeration in Table 30.

**Table 29**

| Number of days of standing | Time for solution (min) in aqueous 35 wt% HCl soln. | Time for solution (min) in ethanol |
|---|---|---|
| 0 | 2.5 | 32 |
| 7 | 2.6 | 34 |
| 14 | 2.6 | 33 |
| 30 | 2.5 | 32 |

**Table 30**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Control 5

In vials having the entrapped air therein displaced with nitrogen gas, samples of the same white crystalline cysteamine hydrochloride powder as obtained in Example 11 were left standing at 30°C for varying lengths of time, i.e. 0 to 30 days, and tested in their undissolved state for solubility in an aqueous 35 wt% hydrochloric acid solution and ethanol and for susceptibility for agglomeration.

The results of the test for solubility are shown in Table 31 and those of the test for agglomeration in Table 32.

**Table 31**

| Number of days of standing | Time for solution (min) in aqueous 35 wt% HCl soln. | Time for solution (min) in N-ethyl-2-pyrrolidone |
|---|---|---|
| 0 | 2 | 22 |
| 7 | 24 | 68 |
| 14 | 30 | 74 |
| 30 | 36 | 84 |

**Table 32**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Example 12

White solid cysteamine hydrochloride hemispheres 10 to 15 mm in diameter and 5 to 6 mm in height produced by following the procedure of Example 11 were tested for solubility in an aqueous 35 wt% hydrochloric acid solution and for susceptibility to agglomeration.

The results of the test for solubility are shown in Table 33 and those of the test for agglomeration in Table 34.

**Table 33**

| Number of days of standing | Time for solution (min) in aqueous 35 wt% HCl soln. |
|---|---|
| 0 | 5.7 |
| 7 | 5.8 |
| 14 | 5.6 |
| 30 | 5.7 |

**Table 34**

| Number of days of standing | Condition of motion |
|---|---|
| 0 | Absence of cohesion between particles |
| 7 | Absence of cohesion between particles |
| 14 | Absence of cohesion between particles |
| 30 | Absence of cohesion between particles |

### Example 13

Cysteamine hydrochloride powder was granulated by the use of a granulating device constructed as illustrated in Figs. 2 and 3. To be more specific, the cysteamine hydrochloride powder was melted at a temperature of 72°C with a melting device (not shown), kept substantially at this temperature and, at the same time, advanced through the conduit 15 kept at this temperature, and dropped by means of the dropping device 14 illustrated in Fig. 2 onto the belt conveyor 6 kept at a temperature of 25°C thereby cooled and solidified, to produce granules having an average particle diameter in the range of 5 mm. In the meantime, the belt conveyor 6 had the lower side thereof cooled by causing ethylene glycol supplied from the cooling device 9 through the pump to be sprayed onto the lower side with the coolant spraying device 7. The ethylene glycol which had fulfilled its role of cooling the belt conveyor 6 was recovered by the recovering device 8 and returned to the cooling device 9, there to be cooled to the prescribed temperature. To the building 1 and the hopper 16, dry air was supplied through the conduits 17, 18 to keep the relative humidity inside them at 30 %. The granules which were recovered in the hopper 16 had a water content of 0.1 % by weight.

### Control 6

Granules having an average particle diameter of 2 to 5 mm were obtained by following the procedure of Example 1, excepting water was, used as a coolant in the place of ethylene glycol. The granules had a water content of 0.7 % by weight.

### Example 14

The procedure of Example 13 was repeated, except that a belt conveyor 16 of stainless steel having attached fast to the surface thereof through the medium of adhesive agent a tape 0.01 mm in thickness obtained by impregnating glass cloth with polytetrafluoroethylene was used as maintained at a temperature of 20°C. The granules which were recovered in the hopper 26 had a water content of 0.1 % by weight. The belt conveyor showed no discernible sign of corrosion even after continued use.

## Claims

1. A method for the production of granular cysteamine hydrochloride having an average particle diameter in the range of 0.1 to 20 mm, characterised by melting cysteamine hydrochloride powder at a temperature in the range of 68° to 150°C and dropping the resultant molten cysteamine hydrochloride onto a corrosion proof substrate maintained at a temperature in the range of 40° to 10°C, thereby cooling and solidifying the drops of cysteamine hydrochloride.

2. A method as claimed in Claim 1, characterised in that the granulation of the molten cysteamine hydrochloride is effected by cooling and solidification by the use of a plate type dropping granulating device.

3. A method as claimed in Claim 1 or Claim 2, characterised in that the cysteamine hydrochloride is obtained by preparing cysteamine by the reaction of hydrogen sulphide with ethylene imine and causing the cysteamine to react with hydrochloric acid.

4. A method as claimed in Claim 3, characterised in that the cysteamine hydrochloride is obtained by keeping hydrogen sulphide under a pressure in the range of 6 to 10 kg/cm²·G at a temperature in the range of 0° to 10°C in the presence of a solvent and continuously adding ethylene imine thereto for reaction therewith, then heating the resultant reaction production to a temperature in the range of 60° to 70°C thereby effecting separation of hydrogen sulphide therefrom, adding hydrochloric acid thereto, and cooling the resultant mixture to a temperature not exceeding 5°C thereby inducing precipitation of crystals.

5. A method as claimed in Claims 1 or Claim 2, characterised in that the cysteamine hydrochloride powder is obtained by causing the sulphuric ester of 2-aminoethanol to react with carbon disulphide in the presence of an alkali hydrolysing the 2-mercaptothiazoline with hydrochloric acid.

6. A method as claimed in Claim 1 or Claim 2, characterised in that the cysteamine hydrochloride powder is obtained by causing 2-aminoethyl sulphuric ester to react with sodium thiosulphate in the presence of an alkali hydroxide thereby forming S-(2-aminoethyl)thiosulphate and hydrolysing the S-(2-aminoethyl)thiosulphate with hydrochloric acid.

7. A method as claimed in Claim 1 or Claim 2, characterised in that the cysteamine hydrochloride powder is obtained by causing 20-chloroethyl amine hydrochloride to react with 2-mercaptothiazoline in the presence of a solvent thereby forming 2-(2'-aminoethylithio)-thiazolidine hydrochloride and hydrolysing the 2-(2'-aminoethylthio)-thiazolidine hydrochloride.

8. A method as claimed in Claim 1 or Claim 2, characterised in that the cysteamine hydrochloride powder is obtained by causing a 2-dialkylthiazoline to react with water and a halogenated hydroacid.

9. A method as claimed in Claim 1, characterised by granulating cysteamine hydrochloride by melting the cysteamine hydrochloride at a temperature in the range of 68° to 150°C and cooling and solidifying the molten cysteamine hydrochloride at a temperature in the range of 40° to 10°C by the use of a plate type dropping granulating device and effecting the required cooling of the cooling substrate of the plate type dropping granulating device by spraying the lower side of the substrate with a polyhydric alcohol containing solution.

10. A method as claimed in Claim 9, characterised in that the polyhydric alcohol is ethylene glycol.

11. A method as claimed in Claim 9 or Claim 10, characterised by causing the molten cysteamine hydrochloride to fall in drops onto the cooling substrate which is then in the form of a metal plate coated with a fluorine resin.

## Patentansprüche

1. Verfahren zur Herstellung eines granulösen Cysteamin-Hydrochlorids mit einem durchschnittlichen Partikeldurchmesser im Bereich von 0,1 bis 20 mm, gekennzeichnet durch das Schmelzen von Cysteamin-Hydrochloridpulver bei einer Temperatur im Bereich von 68°C bis 150°C und Tropfen des erhaltenen geschmolzenen Cysteamin-Hydrochlorids auf ein korrisionsbeständiges Substrat, das bei einer Temperatur im Bereich von 40°C bis 10°C gehalten wird, wodurch die Cysteamin-Hydrochloridtropfen abgekühlt und verfestigt werden.

2. Verfahren nach Anspruch 1, dadurch gekennzeichnet, daß die Granulierung des geschmolzenen Cysteamin-Hydrochlorids durch Abkühlen und Verfestigen unter Verwendung einer plattenartigen Tropfgranulierungsvorrichtung erzielt wird.

3. Verfahren nach Anspruch 1 oder Anspruch 2, dadurch gekennzeichnet, daß das Cysteamin-Hydrochlorid dadurch erhalten wird, daß Cysteamin durch Reaktion von Schwefelwasserstoff mit Ethylenimin hergestellt und die Reaktion des Cysteamins mit Salzsäure herbeigeführt wird.

4. Verfahren nach Anspruch 3, dadurch gekennzeichnet, daß das Cysteamin-Hydrochlorid dadurch erhalten wird, daß Schwefelwasserstoff unter einem Druck im Bereich von 6 bis 10 kg/cm²·G bei einer Temperatur im Bereich von 0°C bis 10°C in Gegenwart eines Lösungsmittels gehalten wird und kontinuierlich Ethylenimin zur Reaktion mit diesem zugegeben wird, dann das erhaltene Reaktionsprodukt auf eine Temperatur im Bereich von 60°C bis 70°C erwärmt wird, um dadurch die Abtrennung des Schwefelwasserstoffes herbeizuführen, dazu Salzsäure zugegeben und die erhaltene Mischung auf eine Temperatur von nicht mehr als 5°C gekühlt wird, wodurch die Ausfällung von Kristallen eingeleitet wird.

5. Verfahren nach Anspruch 1 oder Anspruch 2, dadurch gekennzeichnet, daß das Cysteamin-Hydrochloridpulver dadurch erhalten wird, daß der Schwefelester von 2-Aminoethanol mit Schwefelkohlenstoff in Gegenwart einer Lauge zur Reaktion gebracht und das 2-Mercaptothiazolin mit Salzsäure hydrolysiert wird.

6. Verfahren nach Anspruch 1 oder Anspruch 2, dadurch gekennzeichnet, daß das Cysteamin-Hydrochloridpulver dadurch erhalten wird, daß 2-Aminoethylschwefelester mit Natriumthiosulphat in Gegenwart eines Alkalihydroxids zur Reaktion gebracht wird, wobei S-(2-Aminoethyl)thiosulphat gebildet wird, und das S-(2-Aminoethyl)thiosulphat mit Salzsäure hydrolysiert wird.

7. Verfahren nach Anspruch 1 oder Anspruch 2, dadurch gekennzeichnet, daß das Cysteamin-Hydrochloridpulver dadurch erhalten wird, daß 20-Chloroethylaminhydrochlorid mit 2-Mercaptothiazolin in Gegenwart eines Lösungsmittels zur Reaktion gebracht wird wobei 2-(2'-Aminoethylthio)-thiazolidinhydrochlorid gebildet wird, und das 2-(2'-Aminoethylthio)-thiazolidinhydrochlorid mit Salzsäure hydrolysiert wird.

8. Verfahren nach Anspruch 1 oder Anspruch 2, dadurch gekennzeichnet, daß das Cysteamin-Hydrochloridpulver dadurch erhalten wird, daß ein 2-Dialkylthiazolin mit Wasser und einer halogenierten Hydrosäure zur Reaktion gebracht wird.

9. Verfahren nach Anspruch 1, gekennzeichnet durch das Granulieren von Cysteamin Hydrochlorid durch Schmelzen von Cysteamin Hydrochlorid bei einer Temperatur im Bereich von 68°C bis 150°C und Abkühlen und Verfestigen des geschmolzenen Cysteamin-Hydrochlorids bei einer Temperatur im Bereich von 40°C bis 10°C unter Verwendung einer plattenartigen Tropfgranulierungsvorrichtung und Erzielen der erforderlichen Kühlung des Kühlungssubstrates der plattenartigen Tropfgranulierungsvorrichtung durch Besprühen der Unterseite des Substrates mit einer polyalkoholhaltigen Lösung.

10. Verfahren nach Anspruch 9, dadurch gekennzeichnet, daß der Polyalkohol Ethylenglycol ist.

11. Verfahren nach Anspruch 9 oder Anspruch 10, dadurch gekennzeichnet, daß das geschmolzene Cysteamin Hydrochlorid in Tropfen auf das Kühlungssubstrat fallen gelassen wird, welches dann die Form einer Metallplatte aufweist, die mit einem Fluorharz beschichtet ist.

## Revendications

1. Procédé de préparation de chlorhydrate de cystéamine en grains, ayant un diamètre particulaire moyen dans l'intervalle de 0,1 à 20 mm, caractérisé par la fusion de chlorhydrate de cystéamine en poudre à une température dans l'intervalle de 68° à 150°C et la chute goutte à goutte du chlorhydrate de cystéamine fondu résultant sur un substrat résistant à la corrosion, maintenu à une température dans l'intervalle de 40° à 10°C, pour refroidir et solidifier les gouttes de chlorhydrate de cystéamine.

2. Procédé suivant la revendication 1, caractérisé en ce que la granulation du chlorhydrate de cystéamine fondu est réalisée par refroidissement et solidification au moyen d'un dispositif de granulation par chute de type plaque.

3. Procédé suivant la revendication 1 ou 2, caractérisé en ce que le chlorhydrate de cystéamine est obtenu en préparant de la cystéamine par la réaction de sulfure d'hydrogène avec de l'éthylèneimine et en faisant réagir la cystéamine avec du chlorure d'hydrogène.

4. Procédé suivant la revendication 3, caractérisé en ce que le chlorhydrate de cystéamine est obtenu en maintenant du sulfure d'hydrogène sous une pression dans l'intervalle de 6 à 10 kg par cm².G, à une température dans l'intervalle de 0° à 10°C, en présence d'un solvant et en additionnant de manière continue de l'éthylèneimine à celui-ci pour réagir avec celui-ci, en chauffant ensuite le produit de la réaction résultant à une température dans l'intervalle de 60° à 70°C pour réaliser la séparation du sulfure d'hydrogène, en additionnant à celui-ci de l'acide chlorhydrique et en refroidissant le mélange résultant jusqu'à une température n'excédant pas 5°C pour induire la précipitation des cristaux.

5. Procédé suivant la revendication 1 ou 2, caractérisé en ce que le chlorhydrate de cystéamine en poudre est obtenu par réaction de l'ester sulfurique du 2-aminoéthanol avec du disulfure de carbone, en présence d'un alcalin et par hydrolyse de la 2-mercaptothiazoline par de l'acide chlorhydrique.

6. Procédé suivant la revendication 1 ou 2, caractérisé en ce que le chlorhydrate de cystéamine en poudre est obtenu par réaction de l'ester 2-aminoéthylsulfurique avec du thiosulfate de sodium, en présence d'un hydroxyde alcalin pour former le S-(2-aminoéthyl)thiosulfate et par hydrolyse du S-(2-aminoéthyl)thiosulfate par de l'acide chlorhydrique.

7. Procédé suivant la revendication 1 ou 2, caractérisé en ce que le chlorhydrate de cystéamine en poudre est obtenu par réaction du chlorhydrate de 2-chloroéthylamine avec la 2-mercaptothiazoline en présence d'un solvant, pour former le chlorhydrate de 2-(2'-aminoéthylthio)thiazolidine et par hydrolyse du chlorhydrate de 2-(2'-aminoéthylthio)thiazolidine.

8. Procédé suivant la revendication 1 ou 2, caractérisé en ce que le chlorhydrate de cystéamine en poudre est obtenu par réaction d'une 2-dialcoylthiazoline avec de l'eau et un hydracide halogéné.

9. Procédé suivant la revendication 1, caractérisé par la granulation du chlorhydrate de cystéamine par fusion du chlorhydrate de cystéamine à une température dans l'intervalle de 68° à 150°C et par refroidissement et solidification du chlorhydrate de cystéamine fondu à une température dans l'intervalle de 40° à 10°C au moyen d'un dispositif de granulation par chute de type plaque et réalisation du refroidissement nécessaire du substrat de refroidissement du dispositif de granulation par chute de type plaque par vaporisation du côté inférieur du substrat au moyen d'une solution contenant un alcool polyhydroxylé.

10. Procédé suivant la revendication 9, caractérisé en ce que l'alcool polyhydroxylé est l'éthylèneglycol.

11. Procédé suivant la revendication 9 ou 10, caractérisé par la chute de gouttes du chlorhydrate de cystéamine fondu sur le substrat de refroidissement qui a, alors, la forme d'une plaque métallique enduite d'une résine fluorée.
